Europäisches Patentamt

European Patent Office

Office européen des brevets

(11) Publication number: **0 452 503 A1**

# EUROPEAN PATENT APPLICATION
## published in accordance with Art. 158(3) EPC

(21) Application number: **90916058.2**

(22) Date of filing: **02.11.90**

(86) International application number:
**PCT/JP90/01412**

(87) International publication number:
**WO 91/06857 (16.05.91 91/11)**

(51) Int. Cl.⁵: **G01N 33/53**, G01N 33/577, G01N 33/543

(30) Priority: **02.11.89 JP 284919/89**
**02.11.89 JP 284920/89**

(43) Date of publication of application:
**23.10.91 Bulletin 91/43**

(84) Designated Contracting States:
**AT BE CH DE ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **MATSUZAWA, Kimihiko**
**1-4-24-204, Iguchi Nishi-ku**
**Hiroshima-shi Hiroshima 733(JP)**
Inventor: **HASEGAWA, Ryoichi**
**1-28-5-302, Ozu-cho Iwakuni-shi**
**Yamaguchi 740(JP)**

(74) Representative: **Kraus, Walter, Dr. et al**
**Patentanwälte Kraus, Weisert & Partner**
**Thomas-Wimmer-Ring 15**
**W-8000 München 22(DE)**

(54) **IMMUNOASSAY OF HUMAN THROMBOMODULIN, AND REAGENT AND KIT THEREFOR.**

(57) A method of immunoassaying human thrombomodulin in a specimen by the sandwich technique, wherein one of an antibody (first antibody) immobilized on an insoluble carrier and a labeled antibody (second antibody) is a polyclonal antibody which recognizes human thrombomodulin while the other of them is a monoclonal antibody which recognizes human thrombomodulin.

EP 0 452 503 A1

Fig. 1

1. Scope of the claim for Patent

a. Industrial Utilization

This invention relates to the immunological assay of human thrombomodulin in a sample, especially in a human specimen. More detailedly, this invention relates to a method for immunologically assaying human thrombomodulin existing in a slight amount in a sample with high sensitivity, accurately and easily, a reagent therefor and a kit therefor.

b. Prior Art

Thrombomodulin was found by C. T. Esmon et al. in 1981 from the vascular endothelial cells of a rabbit lung as a cofactor protein remarkably promoting the activation of protein C by thrombin [C. T. Esmon, W. G. Owen: Proc. Natl. Acad. Sci. USA, 78, 2249-2252 (1981)], and purified on the next year [N. L. Esmon, W. G. Owen, C. T. Esmon: J. Biol, Chem., 257, 859-864 (1982)]. Thereafter, human thrombomodulin was also purified from the lung and the placenta [S. Kurosawa et al., Thromb. Res. 37, 353-364 (1985), etc.] and it was reported that thrombomodulin from the human placenta was a glycoprotein having a molecular weight of 105,000 in the presence of the reducing agent and 75,000 in the absence thereof. It is known that thrombomodulin is a membranous protein existing on the cell membrane of the endothelial cells of arterias, veins, capillary vessels and lymph vessels and exists in almost all the internal organs except the brain, and exists particularly in the lung and placenta in a large amount. Further, it is known for its physiological action that it is a physiologically active substance to convert the blood coagulation promotion action of thrombin to a coagulation inhibition action on the endothelial cell of the vessel. Namely, it is known that thrombomodulin forms a 1 : 1 complex with thrombin on the cell membrane and as a result thrombin loses its property as a blood coagulation factor and at the same time manifests a protein C activity promotion action, and exhibits a coagulation inhibition action by inactivating through this activated protein C the active type V factor and the active type VIII factor in the blood coagulation factor. Further, it is also known that thrombomodulin inhibits thrombin formation from prothrombin by directly binding to the active type X factor to inhibit its activity.

Thrombomodulin having such physiological actions as mentioned above has drawn attention as a novel regulatory factor of the blood coagulation-fibrinolysis system. Therefore, it has very great meanings in the studies of the fundamental medical science and clinical medical science of the circulatory system to assay readily and accurately a slight amount of thrombomodulin in a solution.

Particularly, it has a possibility capable of monitoring the pathema of various diseases accompanied by the disorder of the blood vessel wall to assay with high sensitivity human thrombomodulin existing in a slight amount in a human specimen.

Recently, as a method for assaying slight amount components, in a sample, especially in a human specimen is used an immunological assay method utilizing an antigen-antibody reaction. In case of using this assay method as a diagnostic drug in the assay of many specimens in a medical scene, the following points can be mentioned as main requisites. Namely the requisites are, for example, that it has a sensitivity capable of assaying the trace components in a specimen, that it has specificity to assay solely the assay object, that assay values can be obtained with good reproducibility, and that the assay operations are easy and convenient.

It is the present state of thinks that when the so far known means for the assay of human thrombomodulin are evaluated from the above viewpoint, they are not always sufficiently satisfactory. Namely, although there is a report on radioimmunoassay by H. Ishii et al. (H. Ishii et al., J. Clin. Invest., 76, 2178 (1985), H. Ishii et al., Blood 67, 362 (1986)) and this report discloses a competition method using a polyclonal antibody against human thrombomodulin and 125 I-thrombomodulin, its sensitivity is on the order of 1 to 2.5 ng/ml and the assay takes 19 hours or more, and thus this assay method was an unsatisfactory method for assaying a slight amount of thrombomodulin accurately and conveniently. Further, since a polyclonal antibody alone is used as an antibody, there may arise dangerousness on specificity.

Thereafter, there was made a report on an immunological assay method by a sandwich method. Nishioka et al. reports a method using a polyclonal antibody obtained using as an antigen thrombomodulin purified from the human lung [Junji Nishioka et al., Gists of the 10th Japan Thrombus and Hemostasis Society, page 51, (1987)]. Since this method employs as an antibody a polyclonal antibody alone, a problem is conjectured on the specificity of the polyclonal antibody. Further, Japanese Laid-Open Patent Publication Nos. 45398/1989 and 47391/1989 are published. In these publications a sandwich method is disclosed using two kinds of monoclonal antibodies alone, but according to investigations by the present inventors the assay of human thrombomodulin by a sandwich method using monoclonal antibodies alone was not always sufficiently satisfactory as an assay method to be employed in actual medical scenes. Namely, it was not possible to obtain in a short time a sensitivity necessary to assay a slight amount of thrombomodulin in a sample.

c. Problems to be Solved by the Invention

Thus the present inventors studied for providing an immunological assay method, a reagent therefor and a kit therefor, satisfying the requisites as a diagnostic drug.

The first object of the invention lies in providing an immunological assay mthod, a reagent therefor and a kit therefor, capable of assaying with high sensitivity a slight amount of human thrombomodulin in a sample, especially in a human specimen.

The second object of the invention lies in providing an immunological assay method, a reagent and a kit, usable in actual medical treatments, particularly in clinical scenes and assaying conveniently and in a short time.

Another object of the invention lies in providing an immunological assay method, a reagent therefor and a kit therefor, capable of assaying human thrombomodulin in a sample with as small as possible influence on the specificity of the antibody to be used and by persons to make the assay or assay conditions (time, temperature) stably and with a high sensitivity, influenced.

Still other objects of the invention will further be apparent from the following descriptions.

d. Means for Solving the Problems

Thus according to the studies of the present inventors, it was found that the objects and advantages of the above invention can be accomplished by the following immunological assay method, immunological assay reagent and immunological assay kit for human thrombomodulin.

[I] An immunological assay method for human thrombomodulin

In a method for immunological assay human thrombomodulin in a sample by a sandwich method, the method for the immunological assay of human thrombomodulin wherein one of an antibody immobilized on an insoluble carrier (the first antibody) and a labelled antibody (the second antibody) is a polyclonal antibody to recognize human thrombomodulin and the other of them is a monoclonal antibody to recognize human thrombomodulin.

[II] An immunological assay reagent for human thrombomodulin

In a reagent for immunological assay human thrombomodulin in a sample using a first antibody immobilized on an insoluble carrier and a labelled second antibody, the reagent for the immunological assay wherein one antibody is a polyclonal antibody to recognize human thrombomodulin and the other antibody is a monoclonal antibody to recognize human thrombomodulin.

[III] An immunological assay kit for human thrombomodulin

In a kit for immunological assay human thrombomodulin in a sample, the kit for the immunological assay which comprises
(i) an antibody recognizing human thrombomodulin immobilized on an insoluble solid carrier (the first antibody),
(ii) a labelled antibody recognizing human thrombomodulin (the second antibody), provided that one of the above first antibody and the second antibody is a polyclonal antibody recognizing human thrombomodulin and the other is a monoclonal antibody recognizing human thrombomodulin,
(iii) when the antibody is labelled with an enzyme, a substrate for assaying the enzyme activity and a reaction-discontinuing agent,
(iv) a diluent,
(v) a washing agent, and
(vi) a standard substance.
This invention is further detailedly described below.

4

[A-1] Preparation and purification of antigen

As human thrombomodulin as an antigen for obtaining a polyclonal antibody and a monoclonal antibody used in the invention, those of a natural type extracted from natural materials are used in principle, but those obtained by a protein engineering method or a gene engineering method may also be used so long as they have immunological properties equal to natural type human thrombomodulin. As an example, there can be mentioned its haptens, namely peptides containing the antigen determination site of natural type human thrombomodulin. Further, there can further be mentioned fragments obtained by decomposing human thrombomodulin with a proteolytic enzyme such as, for example, elastase or trypsin.

As materials for obtaining natural type human thrombomodulin, there can, for example, be mentioned human lungs, human blood vessels or cultured human vascular endothelial cells or their culture broths, etc., but human placentas are preferred because they are comparatively easy, to obtain. Separation and purification can be carried out in a combination of conventional protein separation techniques such as, for example, salting out, extraction, centrifugation, ultrafiltration and various chromatographies, and the method disclosed in the above literature of S. Kurosawa et al. is an example thereof.

Using the thus obtained purified human thrombomodulin as an antigen, a polyclonal antibody and a monoclonal antibody can be prepared by a hereinafter described method.

[A-2] Preparation of a polyclonal antibody recognizing human thrombomodulin

A polyclonal antibody recognizing human thrombomodulin used in the invention can be obtained by a method known per se using as an antigen the thus obtained human thrombomodulin or its fragment.

Such a polyclonal antibody can be obtained, for example, by the method described in "Edited by Japan Biochemistry Society, Biochemical Experiment Course, second series, volume 5 pages 1 to 10, Tokyo Kagaku Dojin, 1986".

Although animals to be immunized are not particularly limited so long as they are mammals, goats, rabbits, guinea pigs, rats or mice or the like are preferably mentioned when human thrombomodulin is, for example, used as an antigen.

After immunization, blood is withdrawn from the animal and antiserum is obtained. The obtained antiserum is purified by a combination of conventional methods such as, for example, salting out, extraction, centrifugation, ultrafiltration and various chromatographies to a purified polyclonal antibody.

[A-3] Preparation of a monoclonal antibody recognizing human thrombomodulin

The monoclonal antibody, which is used in the invention and recognizes human thrombomodulin, is prepared by using as an antigen the above human thrombomodulin or a fragment thereof, culturing a hybridoma prepared by the cell fusion method known per se by Köhler and Milstein (G. Köhler and Milstein, Nature (London), 256, 495-497 (1975)) to secrete the monoclonal antibody, and separating it from the culture broth. Namely, a mouse is immunized with human thrombomodulin, and the lymphocytes of this mouse were fused with a mouse myeloma cell to prepare hybridomas. Since the thus obtained hybridomas produce various monoclonal antibodies in accordance with the respective various fused lymphocytes, a hybridoma to produce a desired monoclonal antibody is isolated by cloning as a cloned hybridoma. This cloned hybridoma is cultured in vitro to secrete the monoclonal antibody. The anti-human thrombomodulin monoclonal antibody is separated from this culture supernatant.

In the invention, is is preferred to use a monoclonal antibody which has an inhibition action against the formation of the complex of human thrombomodulin and thrombin and has an inhibition action against the activation of human protein C. Such monoclonal antibodies are known, and there are, for example, the monoclonal antibody disclosed in I. Maruyama et al., J. Biol, Chem. 260, 15432 (1985) and the monoclonal antibody, named TM-A73, disclosed in Japanese Laid-Open Patent Publication No. 45398/1989.

The reason why it is preferred to use a monoclonal antibody having the above inhibition action is that the free human thrombomodulin or its fragments in a sample can directly be recognized and assayed without recognizing the complex of human thrombomodulin and thrombin or its fragments.

[B] Preparation of a first antibody and a second antibody

In the invention, although the above polyclonal antibody and monoclonal antibody are used as the antibody immobilized on an insoluble carrier (first antibody) or a labelled antibody (second antibody), as for their combination, one of them is to be the polyclonal antibody and the other is to be the monoclonal antibody. By this combination, it is possible to manifest at the same time the high affinity which the polyclonal antibody has and the high specificity which the monoclonal antibody has in the immunological assay method for human thrombomodulin. As a result, there can be obtained an immunological assay method which is excellent in sensitivity, specificity and reproducibility and convenient.

However, according to the studies by the present inventors it was revealed that a particularly preferred result can be obtained by the combination that the immobilized antibody (first antibody) is a polyclonal antibody recognizing human thrombomodulin and the labelled antibody (second antibody) is a monoclonal antibody recognizing human thrombomodulin.

[B-1] Preparation of immobilized antibody

An anti-human thrombomodulin antibody immobilized on an insoluble (solid) carrier (first antibody) can be obtained as follows.

As the insoluble solid carrier, a polymer obtainable from nature or its derivative, and a synthesized polymer or its derivative can be mentioned. Examples of the former are polysaccharides and their derivatives such as, for example, cellulose, Sephadex, Sepharose, carboxymethylcellulose, nitrocellulose, cellulose acetate and dextran, and inorganic polymers such as glass and silica gel. Examples of the latter are vinyl polymers such as, for example, polystyrene, polyethylene, polypropylene, ABS, polyvinyl fluoride, polyamine-methyl vinyl ether-maleic acid copolymers and ethylene-maleic acid copolymers; and condensation polymers, for example, polyamides such as 6-nylon and 6,6-nylon, polyesters such as polyethylene terephthalate, amino acid polymers, etc. The shape of the insoluble carrier is not particularly limited, and test tubes, microtiter plates, beads, membranes, etc. are examplified. Examples of the antibody immobilized on the insoluble solid carrier are the antibody molecule of the above anti-human thrombomodulin antibody, its fragments maintaining an antigen binding ability such as, for example $F(ab')_2$, Fab', Fab and Fabc, and derivatives of the antibody molecule or its fragments maintaining an antigen binding ability. Methods of immobilizing these antibodies on an insoluble solid carrier include physical adsorption methods such as, for example, a method which comprises immersing a polystyrene solid carrier in a solution of an anti-human thrombomodulin antibody; ionic bond methods such as, for example, a method wherein is used an ion exchange regin or a solid carrier having a functional group which ionizes such as amino groups, carboxylic acid groups, sulfonic acid groups and phosphoric acid groups; covalent bond methods by a chemical reaction such as, for example, carboxychloride methods, carbodiimide methods, maleic anhydride derivative methods, isocyanate derivative methods, cyan bromide-activated polysaccharide methods, diazo methods, active ester methods, and carrier binding methods by a crosslinking reagent (Used as crosslinking reagents are glutaraldehyde, hexamethylene isocyanate, succinimide, maleimide compounds, etc.); further methods wherein binding is made through a substance which does not have a binding ability to human thrombomodulin but is capable of binding to the anti-human thrombomodulin antibody by a biological reaction, for example a method wherein a solid carrier to which protein A bound is used, etc.

According to the studies by the present inventors, it was further revealed that by using as the insoluble solid carrier one having a mirror-like surface, i.e. one having an extremely smooth surface, nonspecific adsorption is lowered and the assay sensitivity of human thrombomodulin is enhanced.

Heretofore, as insoluble solid carriers for highly sensitive assays, those whose surface had been roughened by polishing and thereby whose surface area has been enlarged have rather been used. Indeed when the surface area is enlarged, a merit arises that the amount of the immobilized antibody is increased, but on the other hand a demerit arises that nonspecific adsorption is also enlarged. As a result of the studies by the present inventors, it was found that, in the assay of human thrombomodulin, a solid carrier having a specular surface such that the center line average roughness (Ra) is 1.5 $\mu$m or less is advantageous for the highly sensitive assay of human thrombomodulin because, in the carrier, although the amount of the immobilized antibody is almost equal to that in case of the solid carrier whose surface was roughened, nonspecific adsorption remarkably reduces. Although such insoluble solids having a specular surface are not limited in the quality of the material and shape, polystyrene beads and glass beads are, for example, mentioned.

The centerline average roughness (Ra) means the value of Ra given by the following formula in microns when from the roughness curve the measuring length portion in the direction of the centerline is pulled out and the centerline of the pulled out portion is designated as X-axis and the direction of vertical times is designated as Y-axis, and the roughness curve is expressed by $y = f(x)$.

6

$$Ra = \frac{1}{\ell} \int_0^\ell /f(x)/d(x)$$

Description is made about this center line roughness (Ra) in JIS B 0601-1982 (Japan) < ANSI B46.1-1979 (USA) and R468-1966 (ISO).

In the following examples of the invention, surface roughness was measured using a surface roughness tester Surfcom® 570A produced by TOKYO SEIMITSU CO., LTD.

[B-2] Preparation of labelled antibody

In the invention, a labelled anti-human thrombomodulin antibody (second antibody) can be obtained as follows.

As an antibody to be labelled, there can be used not only the intact antibody molecule, but its fragment whose antigen binding ability is maintained, for example, Fab', F(ab')$_2$ or Facb fragment, etc.

Such fragments, in case of F(ab')$_2$ and Fab', can be obtained by decomposing the thus obtained polyclonal antibody or monoclonal antibody by a known method, for example with pepsin to form the F(ab')$_2$ fragment or further reducing the F(ab')$_2$ fragment to give the Fab' fragment (II. Nisonoff et al., Arch. Biochem. Biophys., 89, 230 (1960): P. Parham., J. Immunol., 131, 2895 (1983), etc.).

As labelling substances to be used for labelling, radioiostopes are well known, but, in view of industrial production, radioisotopes have disadvantages that there is a fear that they cause environmental pollution, they have a time limit for use and special training of the user is necessary, and therefore enzymes are suitable which do not have such drawbacks and have an amplification action.

As enzymes to be bound to such an anti-human thrombomodulin antibody, there can be exemplified, for example, lysozyme, maleate dehydrogenase, glucose-6-phosphate dehydrogenase, peroxidase, glucose oxidase, alkaline phosphatase, luciferase, β-galactosidase, alcohol dehydrogenase, invertase, etc. The binding in the binding antibody of such an enzyme and the above anti-human thrombomodulin antibody can be carried out by a conventional method such as a glutaraldehyde method, a periodic method or a maleimide method. For example, the binding can be conducted by reacting in a solution the maleimidated antibody on a fragment thereof with an enzyme into which SH groups were introduced. The maleimidation of the antibody or its fragment can be carried out, for example with succinimidyl 4-(N-maleimidomethyl)-cyclohexancarbonate (SMCC), sulfosuccinimidyl 4-(N-maleimidomethyl) cyclohexanecarbonate (sulfo SMCC), succinimidyl m-maleimidobenzoate (MBS), succinimidyl 6-maleimidohexanoate (EMCS) or the like. The introduction of SH groups into the enzyme can be carried out by a known method (for example, refer to Edited by Ishikawa, "Koso Meneki Sokutei-ho" (Enzymeimmunoassay) Igaku-Shoin). For example, the introduction can be carried out by reacting the enzyme with S-acetylmercaptosuccini anhydride (AMSA), N-succinimidyl-3-(2-pyridylthio)propionate (SPDP) or the like.

The number of the labelled enzyme in the thus obtained binding antibody of the enzyme and the antihuman thrombomodulin antibody (the second antibody), i.e. the enzyme-labelled antibody can be determined, for example by the measurement of molecular weight or the measurement of absorbance, enzyme activity or the like. For example, in case where the enzyme is peroxidase, the labelling number can be determined by measuring absorbance at 280 nm originating in the antibody and peroxidase and absorbance at 403 nm originating in peroxidase (E. Ichikawa et al., J. Immunoassay, 4, 209-327 (1983), refer to page 243).

Namely, since the absorbance at 403 nm does not originate in the antibody, but solely in peroxidase, the concentration of peroxidase is calculated from the absorbance. Since the absorbance at 280 nm originates in both antibody and peroxidase, the concentration of the antibody is calculated both from a value obtained by subtracting from the measured absorbance at 280 nm the contribution of peroxidase in the absorbance at 280 nm calculated from the concentration of peroxidase calculated from the absorbance at 403 nm and the molecular extinction coefficient of peroxidase at 280 nm, and from the molecular extinction coefficient of the antibody at 280 nm. The labelling number of peroxidase to the antibody can be determined from the thus obtained concentrations of the peroxidase and the antibody.

The present inventors determined the labelling number of the labelled anti-human thrombomodulin antibody of the invention and studied about the interrelation between the labelling number and the assay sensitivity. As a result, it was revealed that preferred result can be obtained when the labelled antibody is used wherein the enzyme is bound to the intact antibody molecule, or its Fab', F(ab')$_2$ or Facb fragment in a rate of 1.0 molecule or more, particularly 1.5 molecules or more (highly labelled antibody), on an average, of the former per 1 molecule of the latter. In order to obtain an assay sensitivity sufficient to assay human thrombomodulin in a sample, it is preferred that the highly labelled antibody is one wherein the enzyme is bound to the intact antibody molecule, or Fab', F(ab')$_2$ or Facb fragment in a rate of 1.5 molecules or more, particularly 2 molecules or more, on an average, of the former per 1 molecule of the latter.

Such a highly labelled antibody can, for example, be prepared by reacting the above maleimidated antibody or antibody fragment with the enzyme into which SH groups were introduced. As the reaction conditions, there can, for example, be employed reaction conditions such that the mole ratio of the antibody to the enzyme is 1 : 1 or more, preferably 1 : 3 or more, the reaction temperature is 4 to 40°C, preferably 4 to 30°C, the reaction pH is 5.5 to 7.5 and the reaction time is 5 to 48 hours, but the reaction conditions are not limited thereto so long as the highly labelled antibody of the invention can be obtained. The thus obtained highly labelled antibody can be separated and purified from the reaction solution using, for example, gel chromatography.

Using the thus obtained enzyme-labelled antibody of the invention, a slight amount of human thrombomodulin in a sample can accurately be assayed with an assay sensitivity necessary for assaying it.

[C] Immunological assay method

As for a sample, particularly a human specimen in the assay method in the invention, any sample can be used so long as it is liquid containing human thrombomodulin, and there can be mentioned a usual clinical sample such as, for example, blood in the form of serum or plasma, arthrosis fluid, lymph fluid, thymus fluid, ascites, amniotic fluid, cell tissue fluid, bone marrow fluid or urine. Preferred is blood in the form of serum or plasma.

In the immunological assay method of the invention, the first antibody and the second antibody are used in combination, and human thrombomodulin in a sample is assayed by a so-called sandwich method.

Sandwich methods are roughly classified into a one step sandwich method and a two step sandwich method. The one step sandwich method is a method which comprises subjecting to antigen-antibody reaction in the same one reaction system a sample containing human thrombomodulin to be assayed, an antibody immobilized on an insoluble carrier (first antibody) and a labelled antibody (second antibody) to form a complex of the immobilized antibody, the antigen and the labelled antibody, and measuring the amount of the labelled substance after a washing operation. In this connection, it is possible to carry out reaction in the simultaneous existence of the sample (specimen), the immobilized antibody and the labelled antibody, or it is also possible to first react the sample with the immobilized antibody and then carry out reaction with the addition of the labelled antibody. Further, it is also possible to previously react the sample with the labelled antibody and then add the immobilzied antibody. Anyway, what is required is that a complex of the immobilized antibody, the antigen and the labelled antibody is formed before the washing operation. On the other hand, the two step sandwich method is a method which comprises reacting first the sample with the immobilized antibody to form a complex of the immobilized antibody and the antigen, removing the sample and washing the complex, adding the labelled antibody to form a complex of the immobilized antibody, the antigen and the labelled antibody, and then measuring the amount of the labelled substance after a washing operation. According to the invention using the polyclonal antibody and the monoclonal antibody, a slight amount of human thrombomodulin in a sample can be assayed by the one step sandwich method or two step sandwich method with high sensitivity, high specificity and good reproducibility and conveniently.

As for the solvent to be used in the immune reaction, any solvent can be used so long as it has no bad influence on the reaction. Preferred examples thereof are those having a pH of the order of 6.0 to 8.0 such as phosphate buffers, Tris hydrochloride buffers and acetate buffers.

There is no particular limitation about the immune reaction temperature condition in the assay so long as the condition does not denature the property of the proteins as a constituent and does not remarkably inhibit the immune reaction, but in general the reaction is carried out under a temperature condition of 50°C or less, preferably of the order of about 4 to 45°C for a time of the order of about 5 minutes to 5 hours, preferably of about 30 minutes to 3 hours.

[D] Immunological assay kit

In the invention, it is possible to constitute a kit for measuring the amount of human thrombomodulin by a combination of an anti-human thrombomodulin antibody (first antibody) immobilized on an insoluble solid carrier, a labelled anti-human thrombomodulin antibody (second antibody) and other things necessary for the immunological assay.

Other reagents combined together with (i) the first antibody and (ii) the second antibody in the immunological assay kit of the invention include those of the following (iii) to (vi).

(iii) when the labelling is carried out using an enzyme, a substrate for measuring the enzyme activity and a reaction-discontinuing agent,

(iv) a diluent,

(v) a washing agent and

(vi) a standard substance

These are described below, but in general those used in immunological assays may be used.

Substrate for measuring the enzyme activity and reaction-discontinuing agent

In the assay kit and the assay method in the invention, as the substrate and reaction-discontinuing agent used when an enzyme is used as a labelling substance there can be used those usually known in immunological assay in accordance with the kind of the enzyme as a labelling substance. As examples thereof, there can be mentioned as substrates of peroxidase diammonium 2,2'-azino-di-[3-ethylbenzothiazolinesulfonate] (ABTS), O-phenylenediamine (OPD), 3,3' ,5,5'-tetramethylbenzidine (TMB), etc. and as discontinuing agents $H_2SO_4$, HCl, acetic acid, a glycine buffer (pH 10.3), a sodium fluoride solution, etc.

Substrates of alkalinephosphatase include 4-nitrophenyl phosphate, 4-methylumbelliferyl phosphate, NADP, etc.

Substrates of $\beta$-galactosidase include 2-ntirophenyl-$\beta$-D-galactoside, 4-methylumbelliferyl-$\beta$-D-galactoside, etc. and discontinuing agents therefor include 0.1 M $Na_2Co_3$, etc.

Diluent

Any diluent can be used in the assay kit and assay method of the invention so long as it is usually used in immunological assay. Any diluent can be used which does not have any bad influence on the immune reaction, and for example, there can mainly be used those having a pH of the range of 6.0 to 8.0 such as phosphate buffers, Tris hydrochrolide buffers and acetate buffers.

Washing agent

In the invention, washing agents usually used in immune reaction assay can be used as such. However by the present inventors, it was found that the use of a specific surfactant in addition to the aforesaid combination of the first antibody and the second antibody brings about an effect that the immune reaction in the assay of human thrombomodulin is not inhibited and that only the nonspecific adsorption of the substances not involved in the immune reaction and the labelled antibody is inhibited.

In the invention, it is advantageous for high sensitivity assay to make such a surfactant exist in an immune reaction solution or washing solution. When the immune reaction is two-stage reaction, the surfactant can be made to exist in any of the reactions but is preferably made to exist in the reaction of the second stage.

The surfactant to be used in the invention is an amphoteric surfactant and/or a nonionic surfactant having an HLB (Hydraphile Lipophile Balance) value of 16 or more.

Nonionic surfactants having an HLB value of 16 or more include, for example, polyoxyalkylene alkyl aryl ether, polyoxyalkylene alkyl ether, polyoxyalkylene polyhydric alcohol fatty acid ester and polyoxyethylene polyoxypropylene polyol nonionic surfactants having an HLB value of 16, and examples of them are Triton X-305 (HLB 17.3), Triton X-405 (HLB 17.9), Emulgen 950 (HLB 182.), Emulgen 985 (HLB 18.9), Tween 20 (HLB 16.7), Pluronic F 68 (HLB 29), Tetronic 707 (HLB > 20), etc.

As amphoteric surfactants, there can, for example, be mentioned carboxylate salt type (betaine type), sulfonate salt type (sulfobetaine type) and phosphate salt type amphoteric surfactants. Sulfobetaine types sometimes have the problem of carcinogenicity, and their industrial use is not always preferred. preferred are betaine type amphoteric surfactants, particularly those represented by the general formula $R_1R_2R_3NR_4$ wherein $R_1$ represents a $C_5$ to $C_{22}$ alkyl group, $R_2$ and $R_3$ represent $C_1$ to $C_5$ alkyl groups and $R_4$ represents a $C_1$ to $C_5$ alkyl group having a $COO^-$ substituent. More preferred are those wherein $R_2$ and $R_3$ are methyl groups and $R_4$ is $CH_2COO^-$. As a suitable surfactant of this group the one wherein $R_1$ is a lauryl group and which is named "Anhitol 24 B" (trade name) is placed on the market from Kao Corporation.

9

These surfactants can be used alone or in a combination of two or more of them.

The concentration of the surfactant in the washing solution or the like is 1 % by weight or less, preferably 0.0025 to 1 % by weight.

In the invention, even when the surfactant is used in an amount of 0.1 % by weight or less, an excellent washing effect is obtained, foaming is diminished at the time of washing, and as a result such problems are reduced that foaming is intensive, and either the removal of the foam becomes complicated or the removal becomes insufficient and, against the intended, washing becomes insufficient.

As a solvent of the surfactant, any solvent can be used so long as it has no bad influence on the assay, and examples thereof are water, physiological saline or a buffer such as a phosphate buffer.

Standard substance

As a standard substance to be used in the assay kit and assay method of the invention, there can be mentioned a protein or polypeptide having at least one antigen determination site which each of the first antibody and the second antibody recognizes.

One of the most representative standard substances is natural type human thrombomodulin extracted from a natural material or its fragments.

As materials from which natural human thrombomodulin is obtained, there can, for example, be mentioned human placentas, human lungs, human blood vessels, cultured human blood vessel endothelial cells and their culture supernatants, etc., but human placentas are preferred because they are relatively easy to obtain. Separation and purification can be carried out in a combination of usually used protein separation techniques such as, for example, salting out, centrifugation, ultrafiltration and various chromatographies, and one example thereof is the method disclosed in the aforesaid literature of S. Kurosawa, et al.

Further, fragments of human thrombomodulin can be obtained by a method known per se, namely by decomposing the thus obtained natural type human thrombomodulin with a proteolytic enzyme such as, for example, an elastase or trysin, or with a protease-decomposing reagent such as, for example, cyanogen bromide [for example, refer to S. Kurosawa et al., J. Biol. Chem., 262, 2206 (1987)].

Further, the standard substance may also be a polypeptide which was obtained by a protein engineering or gene engineering method and has the above properties, as well as the above natural type one.

Use of protein

According to the studies by the present inventors, it was found that when, in the assay of human thrombomodulin, a protein having a molecular weight of 16,000 to 50,000 and an isoelectric point of 1.0 to 5.0 or a mixture containing the same is made to exist in the immune reaction solution and adjustment is made so that the final concentration thereof in the immune reaction solution becomes 0.005 to 0.8 % by weight, nonspecific adsorption is inhibited, and therefore background is remarkably lowered and a high assay sensitivity is obtained. Such a protein or mixture containing it can also be contained in the immune assay reagent or kit of the invention so that the concentration becomes the above predetermined one. Examples of such proteins are casein, pepsin, ovoglycoproteins, orosomucoid, etc. When a protein having a molecular weight of 16,000 or less is used, nonspecific adsorption increases, whereas in the molecular weight of 50,000 or more nonspecific reaction is not sufficiently lowered and specific immune reaction is lowered. Thus, the molecular weight was defined to range from 16,000 to 50,000. Further, as for the isoelectric point, when a protein having an isoelectric point of 5.0 or more, nonspecific adsorption increases, whereas when the isoelectric point is lower than 1.0, specific adsorption is inhibited. Thus, the isoelectric point was defined to be 1.0 to 5.0.

Further, when the immunoassay method was carried out using a solution of the protein, e.g. skim milk in various concentrations, nonspecific reaction strikingly increased at the concentration under 0.005 % by weigh although no antigen existed. Further, when solutions of the protein, e.g. skim milk were preserved in a refrigerator for 1 month, a precipitate incapable of redissolution was formed form the skim milk solutions having a concentration higher than 0.8 % by weight, and thus the concentration was determined to be 0.8 % by weight or less. Taking the two facts into account, the proper concentration of the protein to satisfy the stability of the reagent and effectively reduce the nonspecific reaction is in the range of 0.005 to 0.8 % by weight. Such a mixture can contain, for example, as main components 10 to 60 % by weight, preferably 20 to 50 % by weight of the protein, 30 to 80 % by weight preferably 40 to 60 % by weight of a saccharide (e.g. lactose), and as other components a fat (e.g. 0.5 to 2 % by weight), an ash (e.g. 2 to 8 % by weight), water (e.g. 2 to 8 % by weight), etc. A typical example of such a mixture is skim milk. Although skim milk contains casein as a protein component, skim milk has characteristics of good dispersibility in the immune reaction solution, a high effect in the inhibition of nonspecific reaction and good preservability (a precipitate is hard to form) at 4°C, compared to the case where casein was solely used. The origin of skim milk does not come into question so long as it is defatted milk. The most typical example thereof is skim milk on the market produced by Difco Co.

The "molecular weight" of a protein in the invention means molecular weight measured by the osmotic pressure method. Specifically, this method is a method to measure the melecular weight of a protein utilizing the fact that when a macromolecule solution and a pure solvent are contacted making a semipermeable membrane intervene, between them, which passes freely the solvent molecules but does not pass the dissolved macromolecule, the difference in osmotic pressure of both liquids becomes a parameter of the molecular weight of the macromolecule, and in the invention the measurement was carried out at 4°C using a 6.66 M urea solution. Further, "isoelectric point" means a value measured by the chromatofocusing method wherein a protein is separated according to its isoelectric point, and specifically, means a value measured using a column (0.5 cm∅ x 45 cm) packed with the PBE94 gel (produced by Pharmacia Co.) and 0.025 M imdazole-HCl (pH 7.4) as an eluent.

Although the above protein is used so that its concentration becomes the above concentration in the immune reaction solution, it is preferred, in case of the kit, to make the protein to be contained in at least one of the standard substance, the second antibody and the diluent. The content should appropriately be determined so that the concentration of the protein becomes the above concentration in the immune reaction solution.

In order to obtain further inhibition of non-specific adsorption, namely further lowering of background, in other words, in order to obtain further higher assay sensitivity compared to the case where the protein is solely used, in the assay of human thrombomodulin in the method of the invention, it is preferred to make exist, together with the above protein having a molecular weight of 16,000 to 50,000 and an isoelectric point of 1.0 to 5.0 or the mixture containing it, -globulin or a -globulin derivative in the immune reaction solution in an ultimate concentration of 0.0025 to 0.1 % by weight. Although as -globulin there can be mentioned one separated from the serum of a human being or a mammal such as cattle, a rabbit or a goat, human -globulin particularly preferred. Further, as -globulin derivatives there can be mentioned the above -globulin which was chemically modified, for example S-alkylated -globulin, S-sulfonated -globulin, etc. As for its addition amount, in an amount less than 0.0025 % by weight is small the lowering of background by the addition of -globulin or its derivative, and a substantially sufficient addition effect cannot be obtained. Further, the upper limit of the addition amount is properly 0.1 % by weight from the reason that even if -globulin or its derivative more than 0.1 % by weight is added, an effect higher than that in 0.1 % by weight addition is hardly obtained and from the reason on preparation cost that -globulin is comparatively expensive. From the above reasons, it is proper that the addition amount is in the above range.

e. Effect of the Invention

Thus according to the invention, it is possible to assay with a high sensitivity and a good accuracy and by a convenient procedure an extremely small amount of human thrombomodulin in a sample, for example a clinical sample. Moreover the operation of assay can be made in a comparatively short time.

It is mentioned specifically about the invention that even if the complex of human thrombomodulin and thrombin exists in the sample specimen, the accuracy of the assay of free human thrombomodulin is not influenced by the existence at all. Description of drawings

Figure 1 shows the calibration curve when human thrombomodulin was assayed using the polyclonal antibody and the monoclonal antibody in the invention in comination, and for reference is also shown the calibration curve when antibodies other than those of the invention were combined.

Figure 2 shows the relation between the concentration of the added protein and absorbance in the immunological assay of human thrombomodulin.

Figure 3 shows the relation between the concentration of human thrombomodulin and absorbance (calibration curve).

f. Example

This invention is further specifically described below according to examples.

Example 1

To a rabbit was subcutaneously administered an emulsion of 500 μg of human thrombomodulin purified from the human placenta according to the method disclosed in the above literature of S. Kurosawa et al. with the Freund's complete adjuvant. 14 days later, 250 μg of human thrombomodulin was likewise subcutaneously administered. Human thrombomodulin was further likewise subcutaneously administered twice every 14 days, 10 days thereafter blood was taken, the serum was obtained therefrom, and the antibody value against human thrombomodulin was assayed by the enzyme immunoassay quantitative method, namely using as the antigen human thrombomodulin and as the second antibody the horseradish peroxidase-labelled goat anti-rabbit IgG antibody to confirm that the antibody value is 3 million-fold. Thereafter, the rabbit was exsanguinated, and the serum was separated therefrom and subjected to purification using a protein A-Sepharose-4B column to obtain a polyclonal antibody against human thrombomodulin.

Example 2

1. Maleimidation of the anti-human thrombomodulin monoclonal antibody

13.9 μl of a DMF solution of succinimdyl 4-(N-maleimidomethyl)cyclohexane carbonate (SMCC) (10 mg/ml) was added dropwise at 25°C to a PBS solution of 830 μg of the monoclonal antibody having an inhibition activity against the formation of the complex of human thrombomodulin and thrombin and inhibiting the activation of protein C (TM-A73 disclosed in Japanese Laid-Open Patent Publication No. 45398/1989). After stirring for 30 minutes, the eluate obtained therefrom using a column (1 cm x 45 cm) of Sephadex G-25 was gel filtrated using a 0.1 M phosphate buffer to obtain the maleimidated monoclonal antibody.

2. Introduction of thiol groups into peroxidase

176.4 μl of a DMF solution of S-acetylmercaptosuccinic anhydride (60 mg/ml) was gradually added dropwise to the whole solution of 143mg of peroxidase (Toyobo) in 14.3 ml of 0.1 M phosphate buffer (pH 6.0) under stirring, followed by reaction for 60 minutes. To the resulting solution were added 5.72 ml of a 0.1 M Tris-HCl buffer (pH 7.0), 1.14 ml of a 0.1 M ETDA solution (pH 7.0) and 11.44 ml of a 1 M hydroxylamine solution (pH 7.0), and the mixture was stirred at 30°C for 5 minutes. The resulting solution was placed in a dialysis tube and dialyzed at 4°C for two night using as an outer liquid 0.1 M phosphate butter (pH 6.0) - 5 mM EDTA to obtain a solution of peroxidase into which SH groups are introduced.

3. Linkage of the maleimidated monoclonal antibody to peroxidase into which thiol groups are introduced

830 μg of the maleimidated monoclonal antibody and 1.02 ml of the thiol group-introduced peroxidase solution (4.05 mg/ml) were admixed, the mixture was concentrated to about 600 μl by ultrafiltration, and reaction was carried out at 25°C for 24 hours. The reaction solution was subjected to HPLC (column TSK Gel 3000 SW) and eluted with an eluent PBS (pH 7.2) to obtain a monoclonal antibody to which peroxidase was bound. The mole ratio of the antibody to peroxidase was 1 : 4.6 in the resulting peroxidase-bound monoclonal antibody.

Example 3

The anti-human thrombomodulin polyclonal antibody described in Example 1 was adjusted to a concentration of 5 μg/ml with a 0.1 M carbonate buffer (pH 9.5), and polystyrene beads having a mirror-like surface (Immunochemical: Immunobeads, surface center line average roughness (Ra) measured by Surfcom 570A (produced by TOKYO SEIMITSU CO., LTD.): 1.3 μm) was immersed and allowed to stand at 4°C for 17 hours. The beads were allowed to stand in 1 % BSA-TBS (pH 7.4) at room temperature, and then washed three times with TBS (pH 7.4) to obtain beads A immobilizing the antibody, which was then preserved at 4°C in TBS (pH 7.4) for later use. Beads B immobilizing the anti-human thrombomodulin monoclonal antibody (TM-A73) described in Example 2 were prepared in the same manner as in the above beads A immobilizing the antibody.

On the other hand, solutions containing human thrombomodulin of 0, 0.625, 1.25, 2.5, 5.0 and 10.0 ng/ml respectively were prepared with a diluting buffer 0.5 % BSA - 0.05 % Tween 20 - 5 mM CaCl$_2$ - TBS (pH 7.4). 0.4 ml portions of these solutions were placed in a small plastic test tube made of polypropylene, and a series A of dilutions and a series B of dilutions were prepared each containing human thrombomodulin in concentrations of 0 to 10 ng/ml, respectively. Each one of the beads A immobilizng the antibody was placed in each test tube of the dilution series A, and each one of the beads B immobilizing the antibody was placed in each test tube of the dilution series B, and incubations were carried out at 37° C for 90 minutes, respectively.

The resulting beads were washed three times with a solution of 0.05 % Tween 20 in TBS (pH 7.4) (hereinafter abbreviated as TBS-T); in each test tube of the dilution series A was placed 0.4 ml of a solution obtained by dissolving the peroxidase-labelled anti-human thrombomodulin monoclonal antibody prepared in Example 2 in an antibody concentration of 1 $\mu$g/ml in 0.5 % BSA - 0.05 % Tween 20 - 5 mM $CaCl_2$ - TBS (pH 7.4); in each test tube of the dilution series B was placed 0.4 ml of a solution of an antibody concentration of 4 $\mu$g/ml prepared in the same manner as above using a peroxidase-labelled anti-human thrombomodulin polyclonal antibody prepared in the same manner as in Example 2 from the anti-human thrombomodulin polyclonal antibody described in Example 1; and the resulting mixtures were incubated at 37° C for 30 minutes.

The resulting respective beads were washed five times with TBS-T, 0.4 ml portions of a solution of 2.5 mM $H_2O_2$-0.25 % 3,3',5,5'-tetramethylbenzidine were added thereto respectively, incubation was carried out at 37° C for 30 minutes, the coloring reaction was stopped with the addition of 1 ml of 1 N sulfuric acid, and absorbance was measured at 450 nm.

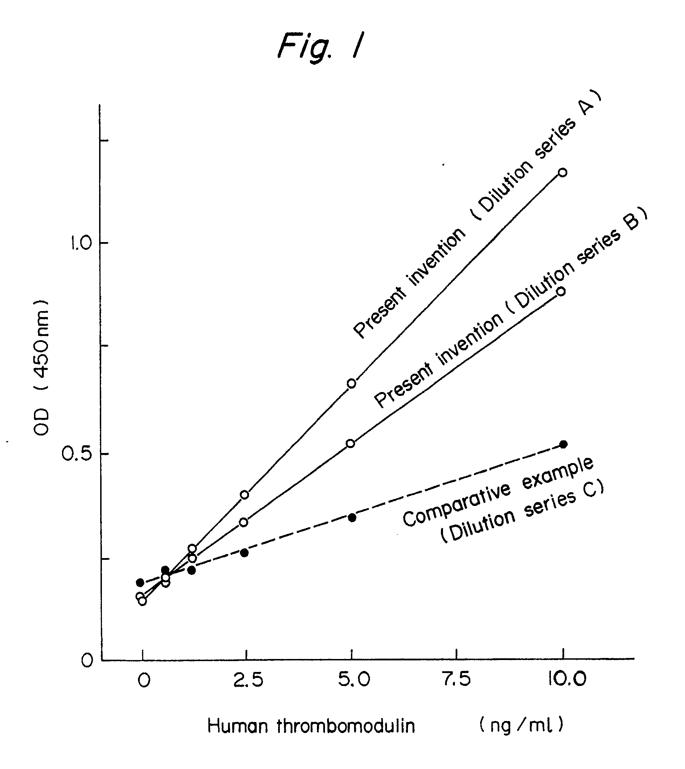

Calibration curves obtained from the results were shown in Figure 1.

For comparison purpose, the monoclonal antibody different from the anti-human thrombomodulin monoclonal antibody of Example 2 in the epitope was immobilized on polystyrene beads having a mirror-like surface in the same manner as in the beads A and B immobilizing antibodies to obtain beads C immobilizing the antibody thereon. A calibration curve on human thrombomodulin (a dilution series C) was obtained in the same manner as that when the beads A immobilizing the antibody were used, except that the beads C immobilizing the enzyme were used. The result was shown in Figure 1 as a comparative example.

As apparent from Figure 1, a calibration curve of a high sensitivity can be obtained in a combination (the dilution series A or the dilution series B) of the polyclonal antibody and the monoclonal antibody of the invention, compared to a combination (the dilution series C) of the two monoclonal antibodies.


Example 4


The anti-human thrombomodulin polyclonal antibody described in Example 1 was dissolved in a 0.1 M carbonate buffer (pH 9.5) so as to give a concentration of 20 $\mu$g/ml, and groups of polystyrene beads for EIA having different center line average roughnesses shown in the following Table 1 were immersed therein and allowed to stand at 4° C for 17 hours. Then, the beads were allowed to stand at room temperature for 2 hours in 1 % BSA-TBS (pH 7.4) and thereafter washed three times with TBS to obtain the various beads immobilizing the antibody.

On the other hand, a 0.17 ng/ml solution of human thrombomodulin was prepared with an diluting buffer 0.5 % BSA - 0.2 % skim milk-0.015 % human $\gamma$-globulin - 15 mM $CaCl_2$ - TBS (pH 7.4), and 0.2 ml portions of the solution were placed in small plastic test tubes made of polypropylene. Further, in each test tube was placed a 0.5 % BSA - 5 mM $CaCl_2$ - TBS (pH 7.4) solution in an antibody concentration of 3 $\mu$g/ml of the peroxidase-labelled anti-human thrombomodulin monoclonal antibody prepared in Example 2, each one of the above groups of beads immobilizing the antibody was placed therein respectively, and the mixtures were incubated at 37° C for 90 minutes. Each bead was washed three times with TBS-T, 0.4 ml of a solution of 2.5 mM $H_2O_2$-0.025 % 3,3',5,5'-tetramethylbenzidine was added thereto as a coloring agent, incubation was carried out at 37° C for 30 minutes, and then the coloring reaction was discontinued with the addition of 1 ml of 1 N sulfuric acid.

Each of the resulting solutions was measured for absorbance at 450 nm, and were calculated the S/N ration of each bead immobilizing the antibody (N is absorbance at human thrombomodulin of 0 ng/ml and S is absorbance at human thrombomodulin of 17 ng/ml) and assay sensitivity (it is supposed that variation coefficient at human thrombomodulin of 0 ng/ml is 10 %, and then assay sensitivity is defined as the human thrombomodulin concentration at the point where the [absorbance + 3 x standard deviation] value at the point 0 obtained from the variation coefficient crosses with the straight line connecting the absorbances at human thrombomodulin concentrations of 0 ng/ml and 17 ng/ml). The results are shown in Table 1.

As apparent from Table 1, it became possible in the invention by the use of an insoluble carrier having a mirror-like surface and Ra of 1.5 $\mu$m or less to assay human thrombomodulin in an excellent sensitivity.

Table 1

| Poly-styrene beads | | Ra | Absorbance at 450 nm | | S/N ratio (measurement sensitivity) |
|---|---|---|---|---|---|
| | | | 0 ng/ml(N) | 17 ng/ml(S) | |
| Present invention | A | μm 0.8 | 0.015 | 0.600 | 40.0 (131 pg/ml) |
| | B | 1.3 | 0.016 | 0.637 | 39.8 (131 pg/ml) |

Example 5

The anti-human thrombomodulin polyclonal antibody described in Example 1 was dissolved in a 0.1 M carbonate buffer (pH 9.5) in a concentration of 5 $\mu$g/ml, and polystyrene beads having a mirror-like surface (produced by Immunochemical Co., Immunobeads, surface center line average roughness (Ra) measured by Surfcom 570A (produced by TOKYO SEIMITSU CO., LTD.): 1.3 $\mu$m) was immersed therein and allowed to stand at 4° C for 17 hours. The beads were washed three times with TBS (pH 7.4) and allowed to stand at room temperature for 2 hours in 1 % BSA-TBS (pH 7.4). The beads were again washed three times with TBS and preserved at 4° C in TBS for later use.

0.10 ng/ml solutions of human thrombomodulin were prepared with solutions of skim milk in ultimate concentrations of 0, 0.005, 0.012, 0.025, 0.05, 0.1 and 0.2 % in 0.5 % BSA - 5 mM CaCl$_2$ - TBS (pH 7.4), and 0.4 ml portions thereof were placed in small plastic test tubes made of polypropylene. In each tube was placed one of the above beads immobilizing human thrombomodulin, and incubation was carried out at 37° C for 90 minutes.

Each bead was washed twice with a solution of 0.05 % Tween 20 in TBS (abbreviated at TBS-T); solutions in an antibody concentration of 1 $\mu$g/ml of the peroxidase-labelled anti-human thrombomodulin monoclonal antibody prepared in Example 2 were prepared with solutions of skim milk in ultimate concentrations of 0, 0.005, 0.012, 0.025, 0.05, 0.1 and 0.2 %, 0.4 ml portions of the prepared solutions were placed in the above tubes corresponding to the same skim milk concentrations respectively; and incubation was carried out at 37° C for 30 minutes.

Each bead was washed three times with TBS-T, 0.4 ml of a solution of 2.5 mM H$_2$O$_2$ - 0.025 % 3,3',5,5'-tetramethylbenzidine was added, incubation was carried out at 37° C for 30 minutes, the coloring reaction was discontinued with the addition of 1 ml of 1 N sulfuric acid, and absorbance at 450 nm was measured.

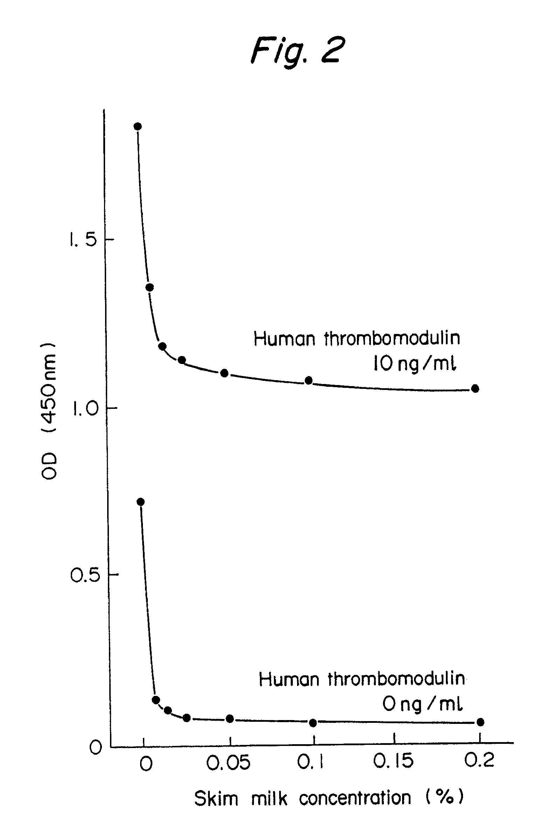

The resulting relation between the skim milk concentration in the immune reaction solution and the absorbance at human thrombomodulin of 0.10 ng/ml was shown in Figure 2. As apparent from the figure, the addition of skim milk inhibits nonspecific adsorption without influencing the specific reaction.

Example 6

Beads immobilizing the anti-human thrombomodulin polyclonal antibody were prepared in the same manner as in Example 5.

On the other hand, solutions in concentrations described in the following Table 2 of human -globulin were prepared with 0.5 % BSA - 0.015 % skim milk - 5 mM CaCl$_2$ - TBS (pH 7.4), and 0 ng/ml and 14 ng/ml solutions of human thrombomodulin were prepared using the above prepared solutions as a diluting buffer. 0.2 ml portions thereof were placed in small test tubes made of polypropylene. Solutions in an antibody concentration of 3 $\mu$g/ml of the peroxidase-lablelled anti-human thrombomodulin monoclonal antibody prepared in Example 2 were prepared with the above diluting buffers, and 0.2 ml portions thereof were placed in the above small test tubes respectively with correspondence in human $\gamma$-globulin concentration.

14

Thereafter, each one of the above beads immobilizing the antibody was placed in each test tube and incubated at 37° C for 90 minutes.

Each bead was washed three times with TBS-T, 0.4 ml of a 2.5 mM $H_2O_2$-0.025 % 3,3',5,5'-tetramethylbenzidine solution was added as a coloring agent, incubation was carried out at 37° C for 30 minutes, the coloring reaction was discontinued with the addition of 1 ml of 1 N sulfuric acid, and absorbance at 450 nm was measured.

From the measured absorbance values, the S/N ratio and assay sensitivity at each human $\gamma$-globulin concentration were calculated in the same manner as in Example 4. The results are shown in Table 2.

It is seen from the results in Table 2 that the assay sensitivity of human thrombomodulin can be enhanced by further adding $\gamma$-globulin in a concentration of 0.0025 % or more to the immune reaction solution containing skim milk.

## Table 2

| Human -globulin concentration | Absorbance at 450 nm | | S/N ratio (Assay sensitivity) |
|---|---|---|---|
| | 0 ng/ml(N) | 14 ng/ml(S) | |
| 0.0025 | 0.040 | 0.643 | 16.1 (278 pg/ml) |
| 0.01 | 0.032 | 0.619 | 19.3 (229 pg/ml) |
| 0.04 | 0.033 | 0.620 | 18.8 (236 pg/ml) |
| 0.10 | 0.034 | 0.631 | 18.6 (239 pg/ml) |

Example 7

In small test tubes made of polypropylene were placed 0.2 ml portions of solutions of human thrombomodulin in concentrations of 0 ng/ml and 17 ng/ml in 0.5 % BSA - 0.2 % skim milk - 0.015 % human -globulin - 5 mM $CaCl_2$ - 50 mM Tris/HCl - 0.1 M NaCl (pH 7.4), and 0.2 ml portions of solutions of the peroxidase-labelled anti-human thrombomodulin monoclonal antibody obtained in Example 2 in an antibody concentration of 3 $\mu$g/ml in 0.5 % BSA - 5 mM $CaCl_2$ - 50 mM Tris/HCl - 0.1 M NaCl (pH 3.4); each one of the beads immobilizing the anti-human thrombomodulin polyclonal antibody prepared in the same manner as in Example 5 was placed in each of the above small test tubes respectively; and incubation was carried out at 37° C for 90 minutes.

The solution in each small test tube was suction removed, and each bead was washed three times with 3 ml each of physiological saline containing 0.05 % the surfactant listed in Table 3. After washing, to each bead was added 0.4 ml of a 2.5 mM $H_2O_2$ - 0.025 % 3,3',5,5'-tetramethylbenzidine solution, incubation was carried out at 37° C for 30 minutes, the coloring reaction was discontinued with the addition of 1 ml of 1 N sulfuric acid, and absorbance at 450 nm was measured. The S/N ratio was calculated from this absorbance in the same manner as in Example 4. The results are shown in Table 3.

Table 3

| | Surfactant | HLB value | Absorbance at 450 nm | | S/N ratio |
| | | | 0 ng/ml(N) | 17 ng/ml(S) | |
|---|---|---|---|---|---|
| Present invention | Lauryl dimethyl-aminoacetate | – | 0.020 | 0.595 | 29.8 |
| | Tetronic 702 | > 20 | 0.021 | 0.712 | 33.9 |
| | Pluronic F68 | 29 | 0.063 | 0.821 | 13.0 |
| | Polyoxyethylene sorbitan mono-laurate (Tween 20) | 16.7 | 0.046 | 0.737 | 16.0 |

Example 8

The same procedure as in Example 7 was carried out using as a washing solution physiological saline solutions of Tetronic 702 in the various concentrations shown in Table 4, whereby the effect of the used concentration was investigated. The results are shown in Table 4.

16

## Table 4

| Tetronic 702 concentration | Absorbance at 450 nm | | S/N ratio |
|---|---|---|---|
| | 0 ng/ml(N) | 17 ng/ml(S) | |
| 0.0025 | 0.062 | 0.710 | 11.5 |
| 0.005 | 0.020 | 0.600 | 30.0 |
| 0.010 | 0.024 | 0.609 | 25.4 |
| 0.050 | 0.018 | 0.542 | 30.1 |
| 0.1 | 0.016 | 0.514 | 32.1 |
| 0.5 | 0.018 | 0.500 | 27.8 |
| 1.0 | 0.015 | 0.472 | 31.5 |

As apparent from the above results, the S/N ratio is large in the range of 0.0025 to 1.0 %, and thus human thrombomodulin can be assayed highly sensitively in the range. At 2 %, foaming is intense, and the absorbance at a human thrombomodulin concentration of 17 ng/ml was strikingly lowered.

Example 9

Beads immobilizing the anti-human thrombomodulin polyclonal antibody were prepared in the same manner as in Example 5.

On the other hand, human thrombomodulin solutions (0 to 33.3 ng/ml) were prepared wherein stepwise dilution was made with 0.5 % BSA - 0.2 % skim milk - 0.015 % human -globulin - 5 mM $CaCl_2$ - 50 mM Tris/HCl - 0.1M NaCl (pH 7.4), and 0.2 ml portions thereof were placed in small test tubes made of polypropylene. Then was prepared a solution of the peroxidase-labelled anti-human thrombomodulin monoclonal antibody prepared in Example 2 in an antibody concentration of 3 $\mu$g/ml in 0.5 % BSA - 5 mM $CaCl_2$ - 50 mM Tris/HCl - 0.1 M NaCl (pH 7.4), and 0.2 ml thereof was placed in each of the above small test tubes. Further, each one of the above beads immobilizing the antibody was placed in each test tube respectively, and incubation was carried out at 37°C for 90 minutes.

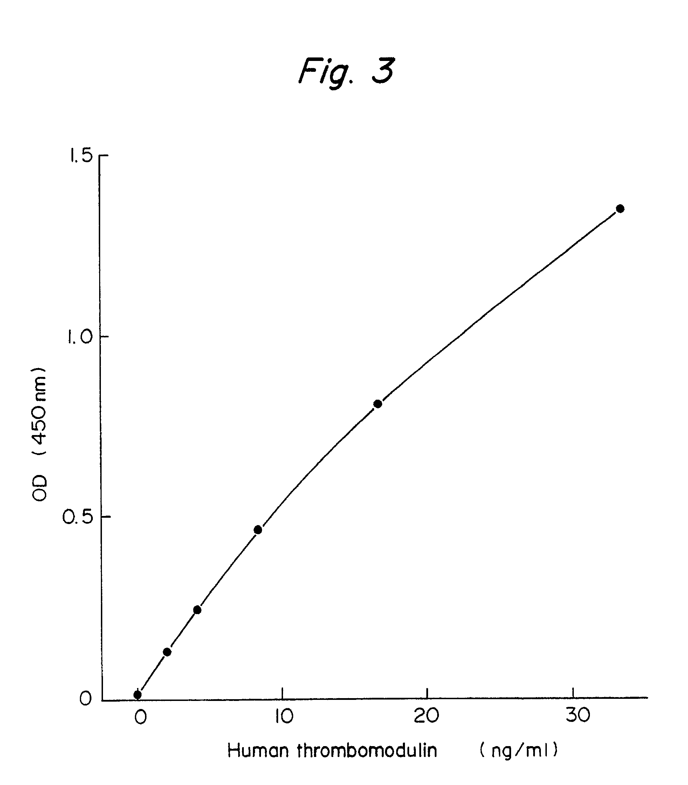

Each bead was washed three times with a 0.005 % Tetronic 702 - 5 mM $CaCl_2$ - physiolosical saline solution, 0.4 ml of a 2.5 mM $H_2O_2$ - 0.025 % 3,3',5,5'-tetramethylbenzidine solution was added as a coloring agent, incubation was carried out at 37°C for 30 minute, the coloring reaction was discontinued with the addition of 1 ml of 1 N sulfuric acid, and absorbance at 450 nm was measured. The resulting calibration curve was shown in Figure 3.

As apparent from Figure 3, a calibration curve having a low background and a high sensitivity (assay sensitivity calculated by the method in Example 4 : 63 pg/ml) can be obtained.

## Claims

1. In a method for immunological assay for human thrombomodulin in a sample by a sandwich method, the method for the immunological assay of human thrombomodulin wherein one of an antibody immobilized on an insoluble carrier (the first antibody) and a labelled antibody (the second antibody) is a polyclonal antibody to recognize human thrombomodulin and the other of them is a monoclonal antibody to recognize human thrombomodulin.

2. The method for the immunological assay of claim 1 wherein the first antibody is the polyclonal antibody to recognize human thrombomodulin and the second antibody is the monoclonal antibody to recognize human thrombomodulin.

3. The method for the immunological assay of claim 1 or 2 wherein the monoclonal antibody is one having an inhibition action on a formation of a complex of human thrombomodulin and human thrombin and having an inhibition action on an activation of human protein c.

4. The method for the immunological assay of any of claims 1 to 3 wherein the insoluble carrier has a specular surface.

5. The method for the immunological assay of any of claims 1 to 4 wherein a protein having a molecular weight of about 16,000 to about 50,000 and an isoelectric point of 1.0 to 5.0 is made to exist in an immune reaction solution in the immunological assay method and the final concentration of the protein in the immune reaction solution is adjusted to 0.005 to 0.8 % by weight.

6. The method for the immunological assay of claims 5 wherein $\gamma$-globulin or a $\gamma$-globulin derivative is made to exist in a concentration of 0.0025 to 0.1 % by weight in the immune reaction solution.

7. The method for the immunological assay of any of claims 1 to 6 wherein a washing agent containing, in a concentration of 0.0025 to 1 % by weight, an amphoteric surfactant, and/or a nonionic surfactant having an HLB value of 16 or more is used as a washing agent in the immunological assay method.

8. In a reagent for immunological assay for human thrombomodulin in a sample using a first antibody immobilized on an insoluble carrier and a labelled second antibody, the reagent for the immunological assay wherein one antibody is a polyclonal antibody to recognize human thrombomodulin and the other of them is a monoclonal antibody to recognize human thrombomodulin.

9. The reagent for the immunological assay of claim 8 wherein the first antibody is the polyclonal antibody to recognize human thrombomodulin and the second antibody is the monoclonal antibody to recognize human thrombomodulin.

10. The reagent for the immunological assay of claim 8 or 9 wherein the monoclonal antibody is one having an inhibition action on a formation of a complex of human thrombomodulin and human thrombin and having an inhibition action on an activation of human protein C.

11. The reagent for the immunological assay of any of claims 8 or 10 wherein the insoluble carrier has a specular surface.

12. In a kit for immunological assay for human thrombomodulin in a sample, the kit for the immunological assay which comprises
    (i) an antibody recognizing human thrombomodulin immobilized on an insoluble solid carrier (the first antibody),
    (ii) a labelled antibody recognizing human thrombomodulin (the second antibody), provided that one of the above first antibody and the second antibody is a polyclonal antibody recognizing human thrombomodulin and the other is a monoclonal antibody recognizing human thrombomodulin,
    (iii) when the antibody is labelled with an enzyme, a substrate for assaying the enzyme activity and a reaction-discontinuing agent,
    (iv) a diluent,
    (v) a washing agent, and
    (vi) a standard substance.

13. The kit for the immunological assay of claim 12 wherein the first antibody is the polyclonal antibody recognizing human thrombomodulin and the second antibody is the monoclonal antibody recognizing human thrombomodulin.

14. The kit for the immunological assay of claims 12 or 13 wherein the monoclonal antibody has an inhibition action on a formation of a complex of human thrombomodulin and human thrombin and has an inhibition action on an activation of human protein C.

15. The kit for the immunological assay of any of claims 12 or 14 wherein the insoluble carrier has a specular surface.

16. The kit for the immunological assay of any of claims 12 or 15 wherein the washing agent contains, in a concentration of 0.0025 to 1 % by weight, an amphoteric surfactant, and/or a nonionic surfactant having an HLB value of 16 or more.

17. The kit for the immunological assay of any of claims 12 or 16 wherein a protein having a molecular weight of about 16,000 to about 50,000 and an isoelectric point of 1.0 to 5.0 is contained in the second antibody, the diluent or the standard substance.

18. The kit for the immunological assay of any of claims 12 or 17 wherein $\gamma$-globulin or a $\gamma$-globulin derivative is contained in the second antibody, the diluent or the standard substance.

# Fig. 1

Fig. 2

*Fig. 3*

# INTERNATIONAL SEARCH REPORT

International Application No PCT/JP90/01412

**I. CLASSIFICATION OF SUBJECT MATTER** (if several classification symbols apply, indicate all) [6]

According to International Patent Classification (IPC) or to both National Classification and IPC

Int. Cl[5] G01N33/53, G01N33/577, G01N33/543

**II. FIELDS SEARCHED**

| Minimum Documentation Searched [7] | |
|---|---|
| Classification System | Classification Symbols |
| IPC | G01N33/53, G01N33/577, G01N33/543 |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, A, 64-45398 (Kowa Co., Ltd.), February 17, 1989 (17. 02. 89), (Family: none) | 1-18 |
| Y | JP, A, 64-47391 (Mitsubishi Gas Chemical Co., Inc.), February 21, 1989 (21. 02. 89), (Family: none) | 1-18 |
| Y | JP, A, 57-16355 (F. Hoffmann-La Roche & Co., AG), January 27, 1982 (27. 01. 82), & BE, A, 888534 & DE, A, 3115115 & GB, A, 2074727 | 1-18 |
| Y | JP, A, 57-500845 (Unilever N.V.), May 13, 1982 (13. 05. 82), & WO, A, 8200058 | 1-18 |
| Y | WO, A, 8901624 (Teijin Ltd.), February 23, 1989 (23. 02. 89), & EP, A, 328679 | 5, 17 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 7, 1991 (07. 01. 91) | January 21, 1991 (21. 01. 91) |
| International Searching Authority | Signature of Authorized Officer |
| Japanese Patent Office | |

Form PCT ISA 210 (second sheet) (January 1985)

| FURTHER INFORMATION CONTINUED FROM THE SECOND SHEET | | |
|---|---|---|
| Y | JP, A, 61-275654 (Teijin Ltd.),<br>December 5, 1986 (05. 12. 86),<br>& WO, A, 8607154 & EP, A, 224590 | 5, 17 |
| Y | JP, A, 63-127161 (Coulter Corp.),<br>May 31, 1988 (31. 05. 88),<br>& EP, A, 260903 & US, A, 4818686 | 5, 17 |
| Y | JP, A, 60-36964 (IQ (BIO) Ltd.),<br>February 26, 1985 (26. 02. 85),<br>& EP, A, 132948 & US, A, 4668639 | 7, 16 |
| Y | JP, A, 60-53846 (Beringer Mannheim GmbH),<br>March 27, 1985 (27. 03. 85), | 7, 16 |

V ☐ **OBSERVATIONS WHERE CERTAIN CLAIMS WERE FOUND UNSEARCHABLE** [1]

This international search report has not been established in respect of certain claims under Article 17(2) (a) for the following reasons:

1. ☐ Claim numbers            because they relate to subject matter not required to be searched by this Authority, namely:

2. ☐ Claim numbers         , because they relate to parts of the international application that do not comply with the prescribed requirements to such an extent that no meaningful international search can be carried out, specifically.

3. ☐ Claim numbers         , because they are dependent claims and are not drafted in accordance with the second and third sentences of PCT Rule 6.4(a).

VI ☐ **OBSERVATIONS WHERE UNITY OF INVENTION IS LACKING** [2]

This International Searching Authority found multiple inventions in this international application as follows.

1. ☐ As all required additional search fees were timely paid by the applicant, this international search report covers all searchable claims of the international application

2. ☐ As only some of the required additional search fees were timely paid by the applicant, this international search report covers only those claims of the international application for which fees were paid, specifically claims:

3. ☐ No required additional search fees were timely paid by the applicant. Consequently, this international search report is restricted to the invention first mentioned in the claims; it is covered by claim numbers:

4. ☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees.

| FURTHER INFORMATION CONTINUED FROM THE SECOND SHEET |
|---|

      & EP, A, 133272 & US, A, 4639425

Y     JP, A, 62-71861 (Beringer Mannheim GmbH),    7, 16
       April 2, 1987 (02. 04. 87),
       & EP, A, 215457

## V. ☐ OBSERVATIONS WHERE CERTAIN CLAIMS WERE FOUND UNSEARCHABLE ¹

This international search report has not been established in respect of certain claims under Article 17(2) (a) for the following reasons:

1. ☐ Claim numbers       because they relate to subject matter not required to be searched by this Authority, namely:

2. ☐ Claim numbers     , because they relate to parts of the international application that do not comply with the prescribed requirements to such an extent that no meaningful international search can be carried out, specifically:

3. ☐ Claim numbers     , because they are dependent claims and are not drafted in accordance with the second and third sentences of PCT Rule 6.4(a).

## VI. ☐ OBSERVATIONS WHERE UNITY OF INVENTION IS LACKING ²

This International Searching Authority found multiple inventions in this international application as follows:

1. ☐ As all required additional search fees were timely paid by the applicant, this international search report covers all searchable claims of the international application

2. ☐ As only some of the required additional search fees were timely paid by the applicant, this international search report covers only those claims of the international application for which fees were paid, specifically claims:

3. ☐ No required additional search fees were timely paid by the applicant. Consequently, this international search report is restricted to the invention first mentioned in the claims; it is covered by claim numbers:

4. ☐ As all searchable claims could be searched without effort justifying an additional fee, the International Searching Authority did not invite payment of any additional fee.

Remark on Protest

  ☐ The additional search fees were accompanied by applicant's protest.

  ☐ No protest accompanied the payment of additional search fees.

Form PCT/ISA/210 (supplemental sheet (2)) (January 1985)